# EUROPÄISCHE PATENTSCHRIFT

(11) **EP 2 830 753 B1**
(45) Veröffentlichungstag und Bekanntmachung des Hinweises auf die Patenterteilung: **26.08.2020**
(21) Anmeldenummer: 13709390.2
(22) Anmeldetag: 06.03.2013
(51) Int. Cl.: C07C 51/06, B01J 19/00, C07C 231/06, C07C 67/08, B01J 4/00, B01J 19/24, C07C 51/377, C07C 51/08, C07C 253/30

(54) **VERFAHREN ZUR HYDROLYSE VON ACETOCYANHYDRIN**
METHOD FOR HYDROLYSING ACETONE CYANOHYDRIN
PROCÉDÉ D'HYDROLYSE D'ACÉTONE-CYANHYDRINE

(30) Priorität: 30.03.2012 DE 102012205257
(43) Veröffentlichungstag der Anmeldung: 04.02.2015
(73) Patentinhaber: Röhm GmbH, 64295 Darmstadt (DE)
(72) Erfinder: SELBACH, Arndt, 50374 Erftstadt (DE); SARTORELLI, Lorenza, 64372 Ober-Ramstadt (DE); PERL, Andreas, 67240 Bobenheim-Roxheim (DE); DEINKEN, Joachim, 65760 Eschborn (DE); SOHNEMANN, Stefanie, 51491 Overath (DE); MNICH, Norbert, 63526 Erlensee (DE); GRÖMPING, Matthias, 64295 Darmstadt (DE); GROPP, Udo, 83093 Bad Endorf (DE); MERTZ, Thomas, 64625 Bensheim (DE)
(74) Vertreter: Röhm Patent Association
(86) Internationale Anmeldenummer: PCT/EP2013/054486
(87) Internationale Veröffentlichungsnummer: WO 2013/143812

(56) Entgegenhaltungen:
- EP-A1- 2 421 637
- US-B2- 7 582 790

## Beschreibung

Die vorliegende Erfindung betrifft ein Verfahren zur Hydrolyse von Acetoncyanhydrin (ACH) mittels Schwefelsäure im Rahmen des ACH-Sulfo-Verfahrens zur Herstellung von Methacrylsäure (MAS) bzw. Methylmethacrylat (MMA).

Die Herstellung von MAS bzw. MMA nach dem ACH-Sulfo-Verfahren ist allgemein bekannt und z.B. in der EP 2054370 beschrieben. Ausgehend von Blausäure und Aceton wird in einem ersten Schritt ACH hergestellt, welches anschließend zum Methacrylamid (MAAm) umgesetzt wird. Diese Schritte sind u. a. in US 7,253,307, EP 1666451 oder EP 2007059092 dargestellt. Zur Herstellung des MAAm wird Acetoncyanhydrin einer Hydrolyse unterzogen. Dabei bildet sich bei verschiedenen Temperaturstufen nach einer Reihe von Reaktionen das gewünschte MAAm. Die Umsetzung wird in einer dem Fachmann bekannten Weise durch eine Reaktion zwischen konzentrierter Schwefelsäure und ACH bewirkt. Die Umsetzung ist exotherm, so dass in vorteilhafter Weise Reaktionswärme aus dem System abgeführt wird.

Die Umsetzung kann im Batchverfahren oder in kontinuierlichen Verfahren durchgeführt werden. Letzteres hat sich in vielen Fällen als vorteilhaft erwiesen. Sofern die Umsetzung im Rahmen eines kontinuierlichen Verfahrens durchgeführt wird, hat sich der Einsatz von Schlaufenreaktoren bewährt. Schlaufenreaktoren sind in der Fachwelt bekannt. Diese können insbesondere in Form von Rohrreaktoren mit Rückführung ausgestaltet sein. Die Umsetzung kann beispielsweise in nur einem Schlaufenreaktor erfolgen. Es kann jedoch vorteilhaft sein, wenn die Umsetzung in einer Kaskade von zwei oder mehreren Schlaufenreaktoren durchgeführt wird.

Ein geeigneter Schlaufenreaktor weist im Rahmen des beschriebenen Verfahrens eine oder mehrere Zufuhrstellen für ACH, eine oder mehrere Zufuhrstellen für konzentrierte Schwefelsäure, einen oder mehrere Gasabscheider, einen oder mehrere Wärmetauscher und einen oder mehrere Mischer auf. Der Schlaufenreaktor kann weitere Bestandteile, wie Fördermittel, Pumpen, Kontrollelemente usw. umfassen.

Die Hydrolyse von ACH mit Schwefelsäure ist exotherm. Parallel zur Hauptreaktion finden mehrere Nebenreaktionen statt, die zur Senkung der Ausbeute führen. In dem bevorzugten Temperaturbereich spielt die Zersetzung von ACH, ebenfalls eine exotherme und schnelle Reaktion, eine wesentliche Rolle. Die im Rahmen der Reaktion anfallende Reaktionswärme muss dem System jedoch zumindest weitgehend entzogen werden, da mit zunehmender Betriebstemperatur und steigender Verweilzeit die Ausbeute sinkt. Grundsätzlich ist es möglich mit entsprechenden Wärmetauschern eine schnelle und umfassende Abfuhr der Reaktionswärme zu erzielen. Es kann jedoch auch nachteilig sein, das Gemisch vor der Dosierung von ACH zu sehr zu kühlen, da sowohl für eine Vermischung als auch für eine effiziente Wärmeabfuhr eine hohe Turbulenz notwendig ist. Da mit sinkender Temperatur die Viskosität des Reaktionsgemisches stark ansteigt, sinkt entsprechend die Strömungsturbulenz, teilweise bis in den Laminarbereich, was im Wärmetauscher zu einer ineffizienteren Wärmeabfuhr und bei der ACH-Dosierung zu einer langsameren und inhomogeneren Mischung führt. Gefordert ist eine schnelle Vermischung von ACH und Reaktionsgemisch, da das ACH reagieren soll bevor es aufgrund der Aufwärmung zersetzt wird.

In der US 7,582,790 z.B. werden Schlaufenreaktoren mit verschiedenen internen Mischaggregaten beansprucht, die aus statischen Mischern, Drosselblenden, Venturi-Düsen, Jet-Düsen, Eduktoren, perforierten Platten, Sprinklern, Dissolvern, Rührmischern, Hochgeschwindigkeits-Schlaufen oder Sprühdüsen bestehen können. Alle diese Mischelemente haben den gemeinsamen Nachteil, dass sie den Strömungswiderstand im Schlaufenreaktor wesentlich erhöhen und zu erheblichen Druckverlusten führen. Letztere bedingen eine verringerte Strömungsgeschwindigkeit und ineffektiveren Wärmeaustausch, was bei dieser tempertursensiblen Reaktion zu Ausbeuteverlusten aufgrund von Temperaturspitzen führt. Ferner findet die Mischung der Reaktionskomponenten in Mischaggregaten statt, die außerhalb der Wärmetauscher angeordnet sind.

Aufgabe der vorliegenden Erfindung ist es daher, die zuvor beschriebenen Nachteile zu beseitigen oder zumindest zu minimieren.

Gelöst wird diese Aufgabe durch ein Verfahren zur Hydrolyse von ACH mittels Schwefelsäure als Vorstufe zur Herstellung von MAS oder MMA in einem Schlaufenreaktor, dadurch gekennzeichnet, dass (a) mindestens einer der im Schlaufenreaktor befindlichen Wärmetauscher mit Turbulatoren ausgestattet ist und (b) mindestens eine der Reaktionskomponenten mittels eines Dosierrings zur Einspeisung mindestens einer der Reaktionskomponenten in den Schlaufenreaktor eingespeist wird wobei der Dosierring zur Einspeisung mindestens einer der Reaktionskomponenten unmittelbar vor einer Pumpe im Schlaufenreaktor eingebaut ist oder Bestandteil der Pumpe und in das Pumpengehäuse integriert ist und der oder die mit Turbulatoren ausgestattete(n) Wärmeaustauscher in Strömungsrichtung nach der Schwefelsäure- und nach der ACH-Zudosierung platziert ist.

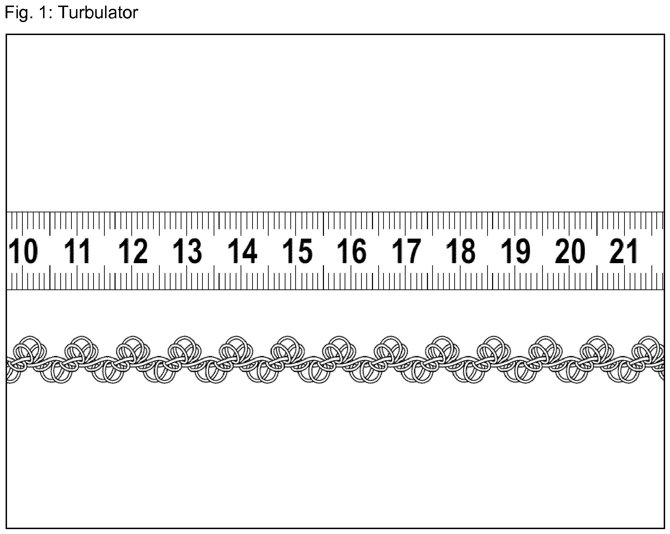

Die erfindungsgemäßen Turbulatoren sind in der EP 061154 beschrieben und werden hiermit offenbart. Sie besitzen den Vorteil, da sie in die Rohre eines Wärmetauschers eingebaut werden, in erster Linie einen guten Wärmeaustausch zu ermöglichen, da sie die Neigung zur Ausbildung einer laminaren Strömung verringern. Die Verwendung dieser Turbulatoren wird z.B. in der WO 2007/075064 für eine heterogene Gasphasenoxidation beschrieben. Überraschend für die Erfinder war, dass diese Turbulatoren trotz ihrer physikalisch einfachen Ausgestaltung (siehe Fig. 1) im vorliegenden komplexen Drei-Phasen-Gemisch mit relativ hoher Viskosität noch einen verbesserten Wärmeaustausch bei gleichzeitig geringem Druckverlust gewährleisten.

Die Turbulatoren werden erfindungsgemäß, wie in EP 061154 beschrieben, in die reaktionsmedium-führenden Rohre eines Rohrbündelwärmeaustauschers eingebaut. Dabei werden mindestens 50-70% der Rohre, bevorzugt 70-90% und besonders bevorzugt 100% der Rohre mit den Turbulatoren ausgestattet. Turbulatoren dieser Art sind z.B. bei der Firma Cal Gavin, Alcester, GB, kommerziell unter der Bezeichnung "hiTRAN Thermal Systems" erhältlich. Die Anzahl der Windungen pro Meter betragen 100-1200 U/m, bevorzugt 300-900 U/m, besonders bevorzugt 500-750 U/m. Die Länge der Turbulatoren ist abhängig von der Länge der Rohre der jeweils eingesetzten Rohrbündelwärmetauscher. Die Turbulatoren sollten die gesamte Länge der Wärmetauscherrohre abdecken.

Neben diesen so definierten Turbulatoren lassen sich auch andere, die Rohröffnung des Rohrbündelwärmetauschers obstruierende Gegenstände einbauen, wie z.B. korkenzieher-ähnliche oder rohrbürsten-artige Strukturen sowie helikalgewundene Metallbänder geeigneter Breite. Ziel dieser Einbauten ist in allen Fällen eine laminare Strömung an der Rohroberfläche zu verhindern und gleichzeitig nur einen minimalen Druckverlust aufzubauen.

Die Platzierung des so ausgestatteten Wärmeaustauschers kann prinzipiell an beliebiger Stelle des Schlaufenreaktors erfolgen, bevorzugt erfolgt sie in Strömungsrichtung jedoch nach der Schwefelsäure- und nach der ACH-Zudosierung.

Die erfindungsgemäßen Dosierringe werden in der EP 2421637 beschrieben und hiermit offenbart. In der dort beschriebenen Verwendung in der vorliegenden ACH-Hydrolyse mittels Schwefelsäure geht man jedoch lediglich vom Stand der Technik mit Anwesenheit von statischen Mischern aus.

Der erfindungsgemäße Dosierring kann verschiedene Ausführungsformen haben. Beispielsweise können viele kleine Dosierstellen in den Ring eingelassen sein, oder wenige große Dosierstellen. Die Dosierstellen können auch über Röhrchen in das Innere des Rohres des Schlaufenreaktors ragen, in besonderen Ausführungsformen auch verschieden lange Röhrchen. Diese besondere Positionierung der Dosierung in von der Rohrwand entfernte Stellen platziert den Reaktionspartner so, dass eine optimale Vermischung in den nachfolgenden Turbulenzen innerhalb der Förderpumpe erfolgt. Die in den Rohrdurchmesser des Schlaufenreaktors hineinragenden Dosierröhrchen können verschiedene Winkel zur Rohrwand einnehmen, bevorzugt einen Winkel ungleich 90 °, besonders bevorzugt in Fließrichtung geneigt. Dies bewirkt, dass der Reaktionspartner gezielt in die inneren Bereiche der Rohrströmung eingebracht und nicht nur an der Rohrwand entlang verteilt wird, letzteres beeinflusst eine schnelle und intensive Mischung durch die Turbulenzen innerhalb der Förderpumpe negativ.

Der Dosierring kann je nach Dosieraufgabe gekühlt oder erwärmt werden. Hier liegt ein weiterer Vorteil des, außen liegenden Ringes, der nicht durch das umgebende Medium erhitzt wird wie im Gegensatz dazu eine Dosierlanze, die zwangsläufig in das Rohr hinein ragt. Eine besondere Ausführungsform ist ein Dosierring, bei dem die Zudosierung unter Überdruck erfolgt. Die Vorrichtung kann jede geeignete Raumform annehmen, bevorzugt ist sie in Ringform konstruiert. Hierbei können auch Doppel- oder Mehrfachringe eingesetzt werden.

Die Edukte können über eine Pumpe in den Rohrreaktor eingeleitet werden. Zur Vermeidung von wartungsbedingten Betriebsunterbrechungen können auch zwei oder mehr Pumpen vorgesehen sein, die parallel geschaltet werden können. Das Mischen der Edukte mit Dosierring kann zweckmäßig in Strömungsrichtung gesehen vor den Pumpen, also auf der Pumpensaugseite, erfolgen. Der Dosierring kann aber auch Bestandteil der Pumpe und in das Pumpengehäuse integriert sein.

Die Komponenten der Anlage, die mit korrosiven Stoffen in Berührung kommen, insbesondere der Rohrreaktor, die Pumpen, Phasentrenner, Dosierring und Wärmetauscher sowie die darin eingebauten Turbulatoren sind aus geeigneten Materialien, beispielsweise einem säureresistenten Metall, wie beispielsweise Zirkonium, Tantal, Titan oder rostfreien Stahl oder einem beschichteten Metall, das beispielsweise eine Emailschicht oder eine Zirkonschicht aufweist, aufgebaut. Weiterhin können auch Kunststoffe, beispielsweise PTFE ummantelte Bauteile, graphitisierte Komponenten oder Werkstücke aus Graphit, insbesondere in Pumpen, eingesetzt werden.

Im Rahmen einer Ausgestaltung des Verfahrens wird aus einem Strom von ACH ein Teil, vorzugsweise etwa zwei Drittel bis etwa drei Viertel, des Volumenstroms in einen ersten Schlaufenreaktor eingeführt. Vorzugsweise weist ein erster Schlaufenreaktor einen oder mehrere Wärmetauscher mit mindestens in einen eingebaute Turbulatoren, eine oder mehrere Pumpen, und einen oder mehrere Gasabscheider auf. Die den ersten Schlaufenreaktor durchlaufenden Umwälzströme liegen vorzugsweise im Bereich von 50-650 m³/h, bevorzugt in einem Bereich von 100-500 m³/h und darüber hinaus bevorzugt in einem Bereich von 150-450 m³/h. In einem auf den ersten Schlaufenreaktor folgenden mindestens einem weiteren Schlaufenreaktor liegen die Umwälzströme vorzugsweise in einem Bereich von 40-650 m³/h, bevorzugt in einem Bereich von 50-500 m³/h und darüber hinaus bevorzugt in einem Bereich von etwa 60-350 m³/h.

Weiterhin sind als Temperaturdifferenz über den Wärmetauscher etwa 1-20 °C bevorzugt, wobei etwa 2-7 °C besonders bevorzugt sind.

Die Zufuhr des ACH, erfindungsgemäß über einen Dosierring, kann grundsätzlich an beliebiger Stelle in den Schlaufenreaktor erfolgen. Es hat sich jedoch als vorteilhaft erwiesen, wenn die Zufuhr unmittelbar an der Ansaugseite einer Pumpe erfolgt. Somit wird die hochturbulente Strömung im Pumpengehäuse zur Mischung der Edukte und dadurch eine Fördermaschine gleichzeitig als zusätzliche Mischmaschine genutzt. Die Zufuhr der Schwefelsäure erfolgt vorteilhafterweise vor der ACH Zugabe. Ansonsten ist es jedoch ebenfalls möglich, die Schwefelsäure an beliebiger Stelle in den Schlaufenreaktor einzuführen.

Das Verhältnis der Reaktanden im Schlaufenreaktor wird so gesteuert, dass ein Überschuss Schwefelsäure vorliegt. Der Überschuss an Schwefelsäure beträgt, bezüglich des molaren Verhältnisses der Inhaltsstoffe, im ersten Schlaufenreaktor etwa 1,8:1 bis etwa 3:1 und im letzten Schlaufenreaktor etwa 1,1:1 bis etwa 2:1.

In einigen Fällen hat es sich als vorteilhaft erwiesen, mit einem derartigen Überschuss an Schwefelsäure die Reaktion im Schlaufenreaktor zu betreiben. Die Schwefelsäure kann hier beispielsweise als Lösemittel dienen und die Viskosität des Reaktionsgemischs niedrig halten, wodurch eine schnellere Abfuhr von Reaktionswärme und eine niedrigere Temperatur des Reaktionsgemischs gewährleistet werden kann. Dies kann deutliche Ausbeutevorteile mit sich bringen. Die Temperatur im Reaktionsgemisch beträgt etwa 85-150 °C.

Die Wärmeabfuhr wird durch einen oder mehrere Wärmetauscher im Schlaufenreaktor gewährleistet. Mindestens einer davon ist mit den erfindungsgemäßen Turbulatoren ausgerüstet. Es hat sich dabei als vorteilhaft erwiesen, wenn die Wärmetauscher über eine geeignete Sensorik zum Einstellen der Kühlleistung verfügen, um eine zu starke Kühlung des Reaktionsgemischs aus den oben genannten Gründen zu verhindern. So kann es beispielsweise vorteilhaft sein, den Wärmeübergang im Wärmetauscher oder in den Wärmetauschern punktförmig oder kontinuierlich zu messen und daran die Kühlleistung der Wärmetauscher anzupassen. Dies kann beispielsweise über das Kühlmittel selbst geschehen. Es ist auch ebenso möglich durch entsprechende Variation der Zugabe der Reaktionspartner und durch die Erzeugung von mehr Reaktionswärme eine entsprechende Erhitzung des Reaktionsgemischs zu erreichen. Auch eine Kombination von beiden Möglichkeiten ist denkbar.

Bevorzugt verfügt der Schlaufenreaktor darüber hinaus über mindestens einen Gasabscheider. Über den Gasabscheider kann einerseits dem Schlaufenreaktor kontinuierlich gebildetes Produkt entnommen werden. Andererseits lassen sich im Rahmen der Reaktion gebildete Gase so dem Reaktionsraum entziehen. Als Gas bildet sich hauptsächlich Kohlenmonoxid. Das aus dem Schlaufenreaktor entnommene Produkt wird vorzugsweise in einen zweiten Schlaufenreaktor überführt. In diesen zweiten Schlaufenreaktor wird das Schwefelsäure und Metharcylsäureamid beinhaltende Reaktionsgemisch, wie es durch die Reaktion im ersten Schlaufenreaktor erhalten wurde, mit dem verbleibenden Teilstrom an ACH umgesetzt. Hierbei reagiert der Überschuss an Schwefelsäure aus dem ersten Schlaufenreaktor, oder zumindest ein Teil der überschüssigen Schwefelsäure, mit dem ACH unter weiterer Bildung von Sulfoxy-Isobuttersäure-Amid (SIBA). Die Durchführung der Reaktion in zwei oder mehr Schlaufenreaktoren weist den Vorteil auf, dass aufgrund des Schwefelsäureüberschusses im ersten Schlaufenreaktor die Pumpbarkeit des Reaktionsgemischs und damit der Wärmeübergang und letztendlich die Ausbeute verbessert werden. Im zweiten Schlaufenreaktor sind wiederum mindestens ein Wärmetauscher und mindestens ein Gasabscheider angeordnet. Ebenso kann der hier dosierte zweite ACH-Teilstrom über einen Dosierring zudosiert werden. Auch mindestens einer der Wärmetauscher im zweiten Schlaufenreaktor ist bevorzugt mit Turbulatoren ausgestattet. Die Reaktionstemperatur im zweiten Schlaufenreaktor beträgt ebenfalls 90-120°C.

Das Problem der Pumpbarkeit des Reaktionsgemischs, des Wärmeübergangs und einer möglichst geringen Reaktionstemperatur stellt sich im jedem weiteren Schlaufenreaktor genauso wie im ersten. Deshalb verfügt vorteilhafter Weise auch der Wärmetauscher im zweiten Schlaufenreaktor über eine entsprechende Sensorik zur Steuerung der Kühlleistung.

Die folgenden Beispiele sollen die Erfindung beschreiben aber in keiner Weise limitieren.

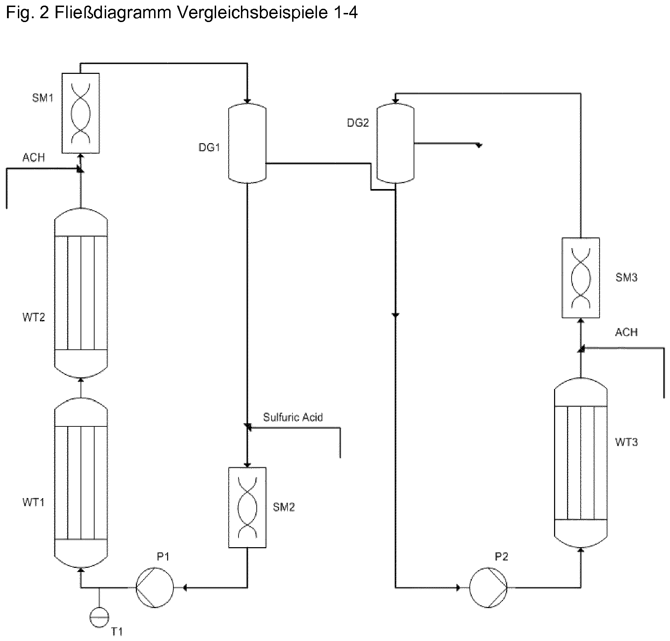

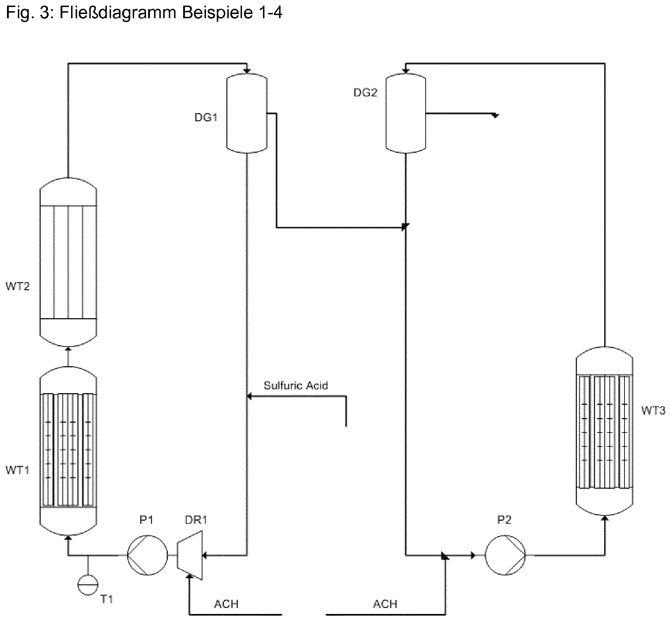

Die Vergleichsbeispiele 1-4 zum Stand der Technik wurden in einer Anlage wie im Fließbild Fig. 2 dargestellt gefahren, die erfindungsgemäßen Beispiele 1-4 analog wie in Fig. 3 dargestellt. In Tab. 1 sind die jeweiligen Prozessparameter und die zugehörigen Ausbeuten aufgelistet.

Fig. 2 zeigt einen zweistufigen Schlaufenreaktor. Etwa Zweidrittel der gesamten ACH-Menge wird vor dem statischen Mischer SM1 in den ersten Kreislauf eindosiert. Die Reaktionslösung durchläuft danach den Entgasungsbehälter DG1. Vor dem statischen Mischer SM2 wird die gesamte Menge an konzentrierter Schwefelsäure zugegeben. Die Pumpe P1 fördert die Reaktionslösung durch zwei Rohrbündelwärmetauscher WT1 und WT2. Am Temperaturmesspunkt T1 wird die Temperatur des ersten Kreislaufes gemessen. Die Reaktionslösung läuft aus dem Entgasungsbehälter DG1 über in den zweiten Kreislauf des Schlaufenreaktors. Die Pumpe P2 fördert die Reaktionslösung durch einen dritten Rohrbündelwärmetauscher WT3. Danach wird die restliche Menge an ACH vor dem statischen Mischer SM3 dosiert. Die Reaktionslösung wird schließlich aus dem zweiten Entgasungsbehälter DG2 in die nächste Verarbeitungsstufe abgeführt.

Fig. 3 zeigt im Prinzip den gleichen Aufbau. Allerdings sind alle statischen Mischer entfernt. ACH wird im ersten Kreislauf unmittelbar vor der Pumpe P1 über den erfindungsgemäßen Dosierring DR1 dosiert. Im Wärmetauscher WT1 sind erfindungsgemäße Turbulatoren mit einer Windungszahl von 550 U/m und einer Länge von 2 m und im Wärmetauscher WT3 solche mit Windungszahl 700 U/m und einer Länge von 1 m eingebaut.

Tab. 1 zeigt die Versuchsergebnisse. Beispiel 1 und Vergleichsbeispiel1 wurden bei mittlerer ACH-Last, niedrigem Molverhältnis Schwefelsäure zu ACH und mittlerem Temperaturniveau in Kreislauf 1 gefahren, Versuchsreihe 2 ebenfalls bei mittlerer ACH-Last, jedoch bei mittlerem Molverhältnis und hohem Temperaturniveau. Versuchsreihe 3 zeigt ebenfalls mittlere ACH-Last und mittleres Molverhältnis, aber niedrigstes Temperaturniveau. Versuchsreihe 4 schließlich wurde bei maximaler ACH-Last, höchstem Molverhältnis und höchstem Temperaturniveau durchgeführt. In allen Versuchsreihen wurde die Temperatur im zweiten Kreislauf, gemessen hinter dem Wärmetauscher WT3, bei ca. 112 °C gehalten.

**Tab. 1: Prozessparameter und Ausbeuten**

| | ACH [kg/h] | H₂SO₄ [kg/h] | T1 [°C] | MV Gesamt | MV Stufe1 | Ausbeute [%] | | Ausbeute [%] |
|---|---|---|---|---|---|---|---|---|
| Beispiel 1 | 4500 | 7255 | 101,8 | 1,41 | 2,2 | 92,2 | Vergleichsbeispiel 1 | 90,5 |
| Beispiel 2 | 4201 | 7013 | 107,1 | 1,46 | 2,2 | 93,2 | Vergleichsbeispiel 2 | 90,0 |
| Beispiel 3 | 4200 | 7013 | 95,4 | 1,46 | 2,2 | 93,4 | Vergleichsbeispiel 3 | 90,4 |
| Beispiel 4 | 6001 | 10364 | 107,1 | 1,51 | 1,8 | 93,3 | Vergleichsbeispiel 4 | 92,1 |

Wie aus Tab. 1 zu ersehen, liegen die Ausbeuten bei den erfindungsgemäßen Beispielen um 2-3 % höher als bei den Vergleichsbeispielen.

### Erläuterungen zu den Figuren:

- SM1, SM2, SM3: Statische Mischer
- WT1, WT2, WT3: Rohrbündelwärmetauscher
- P1, P2: Pumpen
- DR1: Dosierring
- DG1, DG2: Entgasungsbehälter

## Patentansprüche

1. Verfahren zur Hydrolyse von Acetocyanhydrin mittels Schwefelsäure als Vorstufe zur Herstellung von Methylmethacrylat in einem Schlaufenreaktor, **dadurch gekennzeichnet, dass**
a. mindestens einer der im Schlaufenreaktor befindlichen Wärmetauscher mit Turbulatoren ausgestattet ist und
b. mindestens eine der Reaktionskomponenten mittels eines Dosierrings zur Einspeisung mindestens einer der Reaktionskomponenten in den Schlaufenreaktor eingespeist wird
wobei der Dosierring zur Einspeisung mindestens einer der Reaktionskomponenten unmittelbar vor einer Pumpe im Schlaufenreaktor eingebaut ist oder Bestandteil der Pumpe und in das Pumpengehäuse integriert ist und der oder die mit Turbulatoren ausgestattete(n) Wärmeaustauscher in Strömungsrichtung nach der Schwefelsäure- und nach der ACH-Zudosierung platziert ist.

2. Verfahren gemäß Anspruch 1, **dadurch gekennzeichnet, dass** der Dosierring in das Pumpengehäuse integriert ist.

3. Verfahren gemäß Anspruch 1, **dadurch gekennzeichnet, dass** korkenzieher-ähnliche oder rohrbürsten-artige Strukturen als Turbulatoren in mindestens einen Wärmetauscher eingebaut sind.

4. Verfahren gemäß Anspruch 1, **dadurch gekennzeichnet, dass** verschiedenartige Strukturen an Turbulatoren im Schlaufenreaktor verwendet werden.

5. Verfahren gemäß Anspruch 1, **dadurch gekennzeichnet, dass** mindestens 50-70% der Rohre der ausgerüsteten Rohrbündelwärmetauscher mit Turbulatoren bestückt sind.

6. Verfahren gemäß Anspruch 1, **dadurch gekennzeichnet, dass** Turbulatoren mit 500-750 U/m eingesetzt werden.

## Claims

1. Process for hydrolysis of acetone cyanohydrin by means of sulphuric acid as a precursor for preparation of methyl methacrylate in a loop reactor, **characterized in that**
a. at least one of the heat exchangers present in the loop reactor is equipped with turbulators and
b. at least one of the reaction components is fed into the loop reactor by means of a metering ring for feeding in at least one of the reaction components,
wherein the metering ring for feeding in at least one of the reaction components is installed immediately upstream of a pump in the loop reactor or is part of the pump and integrated into the pump housing and the heat exchanger(s) equipped with turbulators is/are positioned downstream of the metered addition of sulphuric acid and of ACH in flow direction.

2. Process according to Claim 1, **characterized in that** the metering ring is integrated into the pump housing.

3. Process according to Claim 1, **characterized in that** corkscrew-like or pipebrush-like structures are installed as turbulators into at least one heat exchanger.

4. Process according to Claim 1, **characterized in that** various kinds of structures are used on turbulators in the loop reactor.

5. Process according to Claim 1, **characterized in that** at least 50-70% of the tubes of the modified shell- and-tube heat exchangers are equipped with turbulators.

6. Process according to Claim 1, **characterized in that** turbulators with 500-750 turns/m are used.

## Revendications

1. Procédé pour l'hydrolyse de la cyanhydrine d'acétone au moyen d'acide sulfurique en tant que précurseur pour la préparation de méthacrylate de méthyle dans un réacteur en boucle, **caractérisé en ce que**
a. au moins un des échangeurs de chaleur se trouvant dans le réacteur en boucle est équipé de turbulateurs et
b. au moins un des composants de réaction est alimenté dans le réacteur en boucle au moyen d'un anneau de dosage pour l'alimentation d'au moins un des composants de réaction
l'anneau de dosage pour l'alimentation d'au moins un des composants de réaction étant installé immédiatement avant une pompe dans le réacteur en boucle ou étant une partie intégrante de la pompe et étant intégré dans le boîtier de la pompe et le ou les échangeur(s) de chaleur équipé(s) de turbulateurs étant placé(s) dans le sens d'écoulement après le dosage de l'acide sulfurique et après le dosage de l'ACH (cyanhydrine d'acétone).

2. Procédé selon la revendication 1, **caractérisé en ce que** l'anneau de dosage est intégré dans le boîtier de la pompe.

3. Procédé selon la revendication 1, **caractérisé en ce que** des structures semblables à un tire-bouchon ou en forme de brosse pour canalisation sont installées en tant que turbulateurs dans au moins un échangeur de chaleur.

4. Procédé selon la revendication 1, **caractérisé en ce que** différentes sortes de structures de turbulateurs sont utilisées dans le réacteur en boucle.

5. Procédé selon la revendication 1, **caractérisé en ce qu'**au moins 50 à 70 % des tubes des échangeurs de chaleur pourvus de faisceau de tubes sont équipés de turbulateurs.

6. Procédé selon la revendication 1, **caractérisé en ce que** des turbulateurs dotés de 500 à 750 U/m sont utilisés.
